# EUROPEAN PATENT SPECIFICATION

(11) **EP 4 084 788 B1**
(45) Date of publication and mention of the grant of the patent: **14.02.2024**
(21) Application number: 21709400.2
(22) Date of filing: 03.03.2021
(51) Int. Cl.: A61K 31/19, A61K 31/194, A61K 31/185, A61K 31/191, A61P 31/12, A61K 33/04, A61P 35/00, A61K 9/00, A61K 9/06, A61K 9/02, A61K 9/10, A61K 9/107, A61K 9/12, A61K 9/14, A61K 47/02, A61K 47/38, A61K 47/12

(54) **COMPOSITION FOR USE IN A TREATMENT OF CERVICAL CELL ABNORMALITIES COMPRISING SELENITE COMPOUND AND ACID**
ZUSAMMENSETZUNG ZUR VERWENDUNG BEI EINER BEHANDLUNG VON ZERVIKALEN ZELLABNORMALITÄTEN MIT SELENITHALTIGER VERBINDUNG UND SÄURE
COMPOSITION DESTINÉE À ÊTRE UTILISÉE DANS UN TRAITEMENT D'ANOMALIES DE CELLULES CERVICALES CONTENANT UN COMPOSÉ DE SÉLÉNITE ET D'ACIDE

(30) Priority: 03.03.2020 EP 20160817
(43) Date of publication of application: 09.11.2022
(73) Proprietor: Selo Medical GmbH, 5585 Unternberg (AT)
(72) Inventor: FUCHS, Norbert, 5580 Unternberg (AT)
(74) Representative: SONN Patentanwälte GmbH & Co KG
(86) International application number: PCT/EP2021/055230
(87) International publication number: WO 2021/175877

(56) References cited:
- EP-A1- 3 616 693
- WO-A1-2012/109685
- DIETMAR SCHMIDT ET AL: "p16/ki-67 dual-stain cytology in the triage of ASCUS and LSIL Papanicolaou cytology : Results from the European Equivocal or Mildly Abnormal Papanicolaou Cytology Study", TARGET PREVALENCE INFLUENCES CYTOLOGISTS' ERROR RATES, vol. 119, no. 3, 25 June 2011 (2011-06-25) , pages 158-166, XP055715456, & 58TH ANNUAL SCIENTIFIC MEETING OF THE AMERICAN-SOCIETY-OF-CYTOPATHOLOGY; BOSTON, MA, USA; NOVEMBER 12 -16, 2010 ISSN: 1934-662X, DOI: 10.1002/cncy.20140
- ALBERTO RAVARINO ET AL: "CINtec PLUS Immunocytochemistry as a Tool for the Cytologic Diagnosis of Glandular Lesions of the Cervix Uteri", AMERICAN JOURNAL OF CLINICAL PATHOLOGY, vol. 138, no. 5, 1 November 2012 (2012-11-01), pages 652-656, XP055715198, US ISSN: 0002-9173, DOI: 10.1309/AJCP00INMGIFYFNQ
- ATTILA LOUIS MAJOR ET AL: "An Adsorptive and Antioxidant Vaginal Gel Clears High-Risk HPV- and p16/Ki-67-Associated Abnormal Cytological Cervical Findings: A post-hoc Subgroup Analysis of a Prospective Randomized Controlled Trial on CIN2 and p16 Positive CIN1", FRONTIERS IN MEDICINE, vol. 8, 25 May 2021 (2021-05-25), XP055946179, DOI: 10.3389/fmed.2021.645559 Retrieved from the Internet: URL:http://dx.doi.org/10.3389/fmed.2021.64 5559>
- Attila Louis Major et al: "Efficacy and safety of an adsorbent and anti-oxidative vaginal gel on CIN1 and2, onhigh-risk HPV, and on p16/Ki-67: a randomized controlled trial", Archives of Gynecology and Obstetrics, 20 November 2020 (2020-11-20), pages 501-511, XP055946180, Retrieved from the Internet: URL:https://link.springer.com/content/pdf/ 10.1007/s00404-020-05816-8.pdf [retrieved on 2022-07-26]

## Description

The present invention relates to a composition for use in a treatment of cervical cell abnormalities.

Cervical smear screening (also known as Pap test or Pap smear) is a widely used practice for detecting potentially precancerous and cancerous processes in the cervix uteri of female patients. Typically, cervical cell abnormalities are classified according to the Bethesda system (see Nayar R, Wilbur D. The Bethesda System for Reporting Cervical Cytology, Definitions, Criteria, and Explanatory Notes. Springer; 2015.). Such abnormalities include atypical squamous cells of undetermined significance (ASC-US), atypical squamous cells - cannot exclude HSIL (ASC-H), atypical glandular cells (AGC), low-grade squamous intraepithelial lesion (LSIL) and high-grade squamous intraepithelial lesion (HSIL), among others. It is known that these abnormalities can improve but also deteriorate with time. In this connection, remission is defined as a complete recovery from abnormal cytological smear findings, regression is defined as an improvement of cytological smear findings (e.g. from HSIL to LSIL or ASC-US, or from LSIL to ASC-US), persistence is defined as no change of cytological smear findings and progression is defined as a worsening of cytological smear findings (e.g. from ASC-US to ASC-H or LSIL, or from ASC-H to LSIL or from LSIL to HSIL).

Depending on the severity of the cervical cell abnormalities, and often after a period of "watchful waiting" to assess whether the abnormalities will remit or regress spontaneously (or, by contrast, will persist or even progress), surgical interventions are recommended according to common treatment guidelines. Typical procedures include cone-shaped excision, cryotherapy, loop electrosurgical excision procedure (LEEP) or large loop excision of the cervical transformation zone (LLETZ).

As any surgical intervention confers a certain health risk, there is a need for non-surgical interventions to reduce the progression rates of cervical cell abnormalities, in order to decrease the need for surgery or at least the invasiveness of the surgery should such an intervention still be necessary.

It is hence an object of the present invention to provide a non-surgical treatment which reduces progression rates of cervical cell abnormalities.

The present invention relates to a pharmaceutical composition containing a selenite-containing compound and a pharmaceutically acceptable acid, selected from citric acid, acetic acid, malic acid, carbonic acid, sulfuric acid, nitric acid, hydrochloric acid, fruit acids (e.g. malic acid, citric acid, tartaric acid, oxalic acid and fumaric acid, in particular citric acid) and mixtures thereof. The composition is for use in reducing progression of cervical cell abnormalities (typically as determined by cervical smear screening) in a female patient. The composition is applied intravaginally to the patient. The patient is p16-positive and Ki-67-positive (i.e. p16/Ki-67-double-positive) at least in a region of the cervix uteri, and/or at least a portion of the cervical cell abnormalities is p16-positive and Ki-67-positive (i.e. p16/Ki-67-double-positive).

In the course of the present invention, it was surprisingly found that the pharmaceutical composition is not only effective in increasing remission and regression of the cervical cell abnormalities, but remarkably also in reducing progression of cervical cell abnormalities. This was confirmed by a controlled clinical trial (see example 2).

While the pharmaceutical composition was previously disclosed for use against other therapeutic indications, these disclosures do not anticipate or suggest the present invention:
US 2003/0180387 A1 relates to a method for increasing the antioxidative potential of selenium-containing aqueous solutions. It is disclosed that a preparation comprising a pharmaceutically administrable or food-compatible form of selenium, namely selenite, and a pharmaceutically acceptable or food-compatible acid, selected from citric acid, acetic acid, malic acid, carbonic acid, various fruit acids and mixtures thereof, can be used to prevent or treat herpes simplex infections, among many other indications. However, the document neither discloses intravaginal application nor that the target organ is the cervix uteri nor the inventive therapeutic indication.

US 2005/0048134 A1 concerns the use of a selenite-containing compound to be topically or buccally administered. Treatment or prophylaxis of infections with papilloma viruses, particularly in the genital region, is disclosed as one among many indications. However, the document neither discloses intravaginal application nor that the target organ is the cervix uteri nor the inventive therapeutic indication.

US 2013/0323328 A1 (also published as WO 2012/109685 A1) relates to a pharmaceutical preparation containing selenite or selenite-containing compounds for treating cervical dysplasia or carcinomas. However, the document is silent at least with respect to the inventive therapeutic indication.

In a particularly preferred embodiment of the present invention, the cervical cell abnormalities to be treated are selected from ASC-US, ASC-H, AGC, LSIL and HSIL, preferably from ASC-US, ASC-H, AGC and LSIL, more preferably from ASC-US, ASC-H and AGC, yet even more preferably from ASC-US and ASC-H, in particular from ASC-US, all according to the Bethesda system (see also explanation and book citation above).

In embodiments, the patient has cervical cell abnormalities selected from ASC-US, ASC-H, AGC, LSIL and HSIL, preferably from ASC-US, ASC-H, AGC and LSIL, more preferably from ASC-US, ASC-H and AGC, yet even more preferably from ASC-US and ASC-H, in particular from ASC-US; in particular as determined by cervical smear screening (especially of said region of the cervix uteri).

According to a preference, said progression of cervical cell abnormalities comprises (or: is further defined as) at least one of change from ASC-US to ASC-H, change from ASC-US to AGC, change from ASC-US to LSIL, change from ASC-US to HSIL, change from ASC-H to AGC, change from ASC-H to LSIL, change from ASC-H to HSIL, change from AGC to LSIL, change from AGC to HSIL and change from LSIL to HSIL, preferably at least one of change from ASC-US to ASC-H, change from ASC-US to AGC, change from ASC-US to LSIL, change from ASC-US to HSIL, change from ASC-H to AGC, change from ASC-H to LSIL and change from ASC-H to HSIL, more preferably at least one of change from ASC-US to ASC-H, change from ASC-US to AGC, change from ASC-US to LSIL and change from ASC-US to HSIL, in particular at least one of change from ASC-US to ASC-H and change from ASC-US to LSIL. Preferably, such a change is determined after at least 30 days, preferably at least 60 days, more preferably at least 90 days, even more preferably at least 120 days, yet even more preferably at least 150 days or even at least 180 days have passed after initial screening which may e.g. performed when starting the inventive treatment.

In the context of the present invention, reducing progression is preferably defined as not comprising increasing remission and increasing regression.

p16 (also known as p16^{INK4a} or cyclin-dependent kinase inhibitor 2A) is a protein that is encoded by the *CDKN2A* gene in humans. Overexpression of p16 is a biomarker of increased cervical cancer risk. Ki-67 (also known as KI-67 or MKI67) is a protein that is encoded by the *MKI67* gene in humans. Ki-67 is a biomarker for cell proliferation. Both of these biomarkers may be tested for instance with the CINtec^{®} PLUS test (Roche, Switzerland) which is a commercially available, approved cytological assay (see e.g. Ravarino, A., et al. (2012). CINtec PLUS immunocytochemistry as a tool for the cytologic diagnosis of glandular lesions of the cervix uteri. American Journal of Clinical Pathology, 138 (5), 652-656). p16/Ki-67 dual-staining is also disclosed in Schmidt, Dietmar, et al. "p16/Ki-67 dual-stain cytology in the triage of ASCUS and LSIL Papanicolaou cytology: results from the European equivocal or mildly abnormal Papanicolaou cytology study." Cancer cytopathology 119.3 (2011): 158-166.

In the course of the present invention, it was found that the inventive therapy is especially effective in reducing progression of cervical cell abnormalities when they are associated with biomarkers p16 and Ki-67. Accordingly, the patient is p16-positive and Ki-67-positive, at least in a region of the cervix uteri (and/or at least a portion of the cervical cell abnormalities is p16-positive and Ki-67-positive). Preferably, the pharmaceutical composition is applied until the patient has become p16-negative, preferably p16-negative and Ki-67-negative, in said region (and/or until at least a portion of the cervical cell abnormalities has become p16-negative and/or Ki-67-negative).

The skilled person knows how to assess whether a region of the cervix uteri (or at least a portion of the cell abnormalities) is p16-positive, e.g. by performing a biopsy in the region and using a p16 assay (such as an immunocytochemistry assay) known in the art. Preferably, a biopsy sample obtained from the region of the cervix uteri is defined as p16-positive if at least one cell, preferably at least two adjoining cells, more preferably at least three adjoining cells, even more preferably at least five adjoining cells, in particular at least ten adjoining cells over-express p16 compared to healthy control tissue, such as healthy adjoining cells in the biopsy sample, preferably wherein each of said cells are epithelial cells. Conversely, such a biopsy sample is preferably defined as p16-negative if less than ten adjoining cells, preferably less than five adjoining cells, more preferably less than three adjoining cells, even more preferably less than two cells, in particular no cell over-expresses p16 compared to healthy control tissue, such as healthy adjoining cells in the biopsy sample, preferably wherein each of said cells are epithelial cells.

Further, the skilled person also knows how to assess whether a region of the cervix uteri (or at least a portion of the cell abnormalities) is Ki-67-positive, e.g. by performing a biopsy in the region and using a Ki-67 assay (such as an immunocytochemistry assay) known in the art. Preferably, a biopsy sample obtained from the region of the cervix uteri is defined as Ki-67-positive if at least one cell, preferably at least two adjoining cells, more preferably at least three adjoining cells, even more preferably at least five adjoining cells, in particular at least ten adjoining cells over-express Ki-67 compared to healthy control tissue, such as healthy adjoining cells in the biopsy sample, preferably wherein each of said cells are epithelial cells. Conversely, such a biopsy sample is preferably defined as Ki-67-negative if less than ten adjoining cells, preferably less than five adjoining cells, more preferably less than three adjoining cells, even more preferably less than two cells, in particular no cell over-expresses Ki-67 compared to healthy control tissue, such as healthy adjoining cells in the biopsy sample, preferably wherein each of said cells are epithelial cells.

In a particular preference, "p16-positive and Ki-67-positive" is defined as a positive result in the established CINtec^{®} PLUS test (Roche, Switzerland), especially as disclosed in CINtec^{®} PLUS Interpretation Guide (Roche, 2016). In this Interpretation Guide, a positive result is defined as the presence of at least one dual-stained cervical epithelial cell (the cytoplasm stains brown (p16) and the nucleus stains red (Ki-67)).

In the course of the present invention, it turned out that the inventive composition is effective already at surprisingly low concentrations of selenium. Therefore, in a further preferred embodiment, the total selenium content of the pharmaceutical composition is 0.01 mg - 1.25 mg, preferably 0.025 mg - 1.00 mg, more preferably 0.05 mg - 0.75 mg, even more preferably 0.10 mg - 0.50 mg, yet even more preferably 0.15 mg - 0.40 mg, especially 0.20 mg - 0.30 mg, per 5 ml of the composition. It is evident that "total selenium content" does not imply that selenium has to be present as elemental selenium in the composition. By way of example, 0.83 mg sodium selenite as the only selenium-containing compound per 5 ml of composition corresponds to a total selenium content ("selenium-equivalent content") of 0.25 mg per 5 ml.

It was also found that the selenium dose per application can be low and still be effective. According to a further preferred embodiment, the total selenium dose is 0.01 mg - 1.25 mg, preferably 0.025 mg - 1.00 mg, more preferably 0.05 mg - 0.75 mg, even more preferably 0.10 mg - 0.50 mg, yet even more preferably 0.15 mg - 0.40 mg, especially 0.20 mg - 0.30 mg, per application. It is evident that "total selenium dose" does not imply that selenium has to be present as elemental selenium in the dose. By way of example, 0.83 mg sodium selenite as the only selenium-containing compound of a dosage unit applied corresponds to a total selenium dose ("selenium-equivalent dose") of 0.25 mg per application.

For increased effectiveness, it is preferred that the pharmaceutical composition is applied at least once per day, preferably for at least 30 days, more preferably for at least 60 days, even more preferably for at least 90 days.

It has turned out that application of the composition only once per day is suitable for achieving effectiveness. As this further reduces the risk of side effects compared to several applications per day, a further preferred embodiment relates to the inventive pharmaceutical composition for use wherein the composition is applied once per day, preferably for at least 30 days, more preferably for at least 60 days, even more preferably for at least 90 days.

The application of the pharmaceutical composition may be discontinued during menstruation of the patient.

The pharmaceutical composition for use according to the present invention may additionally contain further suitable ingredients and/or pharmaceutically acceptable excipients.

Preferably, the pharmaceutical composition for use according to the present invention contains selenite in the form of sodium selenite (which is mostly present as a pentahydrate compound which starts to release crystal water at 40°C).

In a further preferred embodiment of the present invention, the composition contains one or more acids in a total amount between 1 mg and 10 g acid, more preferably between 10 mg and 5 g acid, in particular between 100 mg and l g acid, per 100 g of the composition (in particular if the acid is added in its solid form). Alternatively, the acid may also be added in its liquid form (e. g. with water, i.e. as an aqueous solution). Water and aqueous solutions, respectively, optionally containing further ingredients, may be added to the composition according to the present invention in an amount between 0 and (about) 99.9 g, preferably between 50 and 99 g, in particular between 80 and 98g, per 100 g of the composition.

According to a further preferred embodiment, the composition is present in the form of a gel, a suspension, an emulsion, a suppository such as gelatine capsules or gelatine-free capsules, a spray or a powder.

When present in the form of a gel, the pharmaceutical composition for use according to the present invention preferably contains a gelling agent. Both inorganic and organic aqueous gelling agents may be used as a gelling agent. Particularly suitable gelling agents are cellulose derivatives, in particular carboxymethylcellulose, methylcellulose, hydroxypropyl cellulose and, in particular, hydroxyethyl cellulose. Preferably, the gelling agents, in particular hydroxyethyl cellulose, are used at a total concentration of between 0.1 g and 30 g, more preferably between 0.5 g and 5 g, in particular between 1 g and 3 g, per 100 g of the composition.

A further particularly preferred embodiment of the composition for use according to the present invention, especially when the composition is present in the form of a gel, contains silicon dioxide, in particular highly dispersed silicon dioxide, e. g. according to WO 2001/85852 A1, as a technological suspension medium and/or as an adsorbent. Preferably, an amount between 100 mg and 50 g, more preferably between 500 mg and 10 g, in particular between 1 g and 5 g, SiO2 per 100 g of the composition is used.

The composition for use according to the present invention preferably has a pH-value of less than 7.0, more preferably less than 5.0, in particular between 4.0 and 2.5.

Advantageously, the composition may contain further excipients and/or further active ingredients, in particular buffer substances, colouring agents, stabilizers, preservatives, carrier substances or combinations thereof. Preferred examples of preservatives are potassium sorbate and sodium benzoate.

Moreover, the composition may additionally comprise further active agents such as antibiotics, antiviral agents, antimycotics, pain inhibitors, anti-inflammatory agents or combinations thereof.

The present invention also relates to the composition for use in reducing progression of cervical cell abnormalities in a female patient wherein the patient is p16-positive and Ki-67-positive at least in a region of the cervix uteri, comprising
- obtaining a pharmaceutical composition as defined herein; and
- administering an effective amount of the composition to the patient, wherein the composition is applied intravaginally.

Typically, the patient is in need of the inventive treatment.

According to a particular preference, the human papillomavirus (HPV) infection status of said region of the cervix uteri, preferably of the entire cervix uteri, of the patient is unknown or is not determined. In addition, or alternatively thereto said region of the cervix uteri, preferably the entire cervix uteri, is preferably not infected with any HPV selected from HPV16, HPV18, HPV31, HPV33 and HPV58 (preferably with neither of the five).

HPV infections may be specifically detected e.g. by polymerase chain reaction (PCR) or transcription-mediated amplification (TMA) assays of biopsy samples such as cervical smears, or other methods known in the art, since the genome sequences of HPV16, HPV18, HPV31, HPV33 and HPV58 are known. Genome sequences of these HPV types are for instance published in National Center for Biotechnology Information (NCBI) GenBank under the following accession numbers: K02718.1 (HPV16), X05015.1 (HPV18), J04353.1 (HPV31), M12732.1 (HPV33) and D90400.1 (HPV58).

Herein, the patient to be subject to inventive treatment is preferably older than 20 years, preferably older than 30 years, even more preferably older than 40 years, yet even more preferably older than 50 years. The patient is human.

The inventive treatment has been found to be not as efficient in immunosuppressed patients. Therefore, the patient is preferably not immunosuppressed. In addition, or alternatively thereto, the patient preferably does not have cancer and/or chronic viral disease (or chronic viral disease other than HPV infection).

Herein, the term "over-express" or similar in regard to a gene (product) A typically can mean that the expression level of A (as e.g. measured by Western blot or immunohistochemistry or immunocytochemistry) e.g. in a biopsy sample is higher than the expression level of an appropriate control (such as healthy tissue of the same type), by a factor of at least 1.2 (i.e. an increase of at least 20%), preferably at least 1.4, more preferably at least 1.6, even more preferably at least 1.8, especially at least 2.0.

The present invention is further illustrated by the following figures and examples, without being restricted thereto.

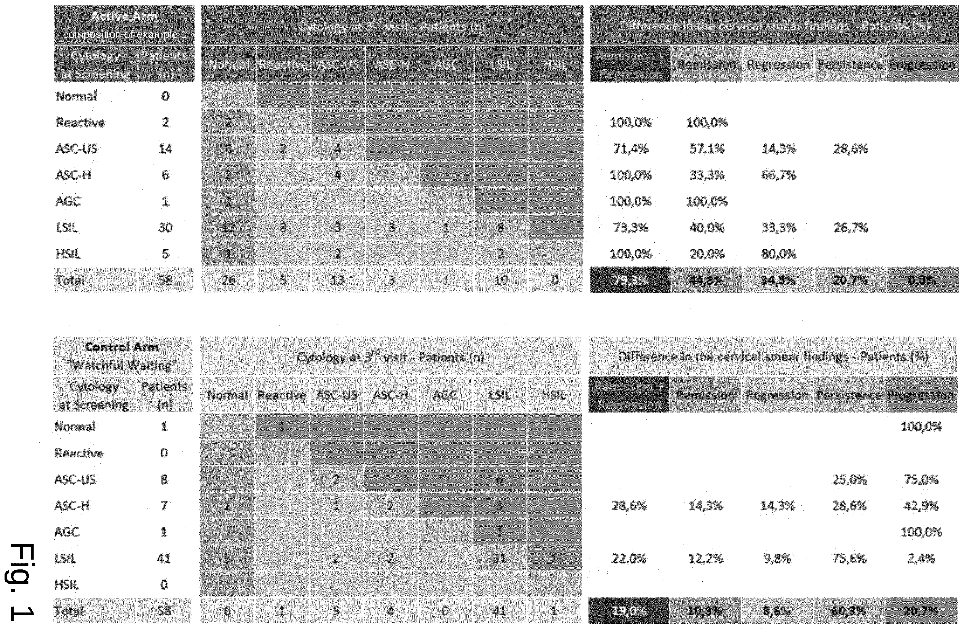

**Fig. 1****: Difference in the cervical smear findings** - **Screening vs. 3^{rd} visit.** An interim analysis showed that compared to the non-treated control arm the progression rate was strongly reduced in the active arm treated with the inventive therapy (0.0% in the active arm vs. 20.7% in the control arm). "at Screening": initial screening of the patient, "at 3^{rd} Visit": after 3x28 days of once-daily treatment.

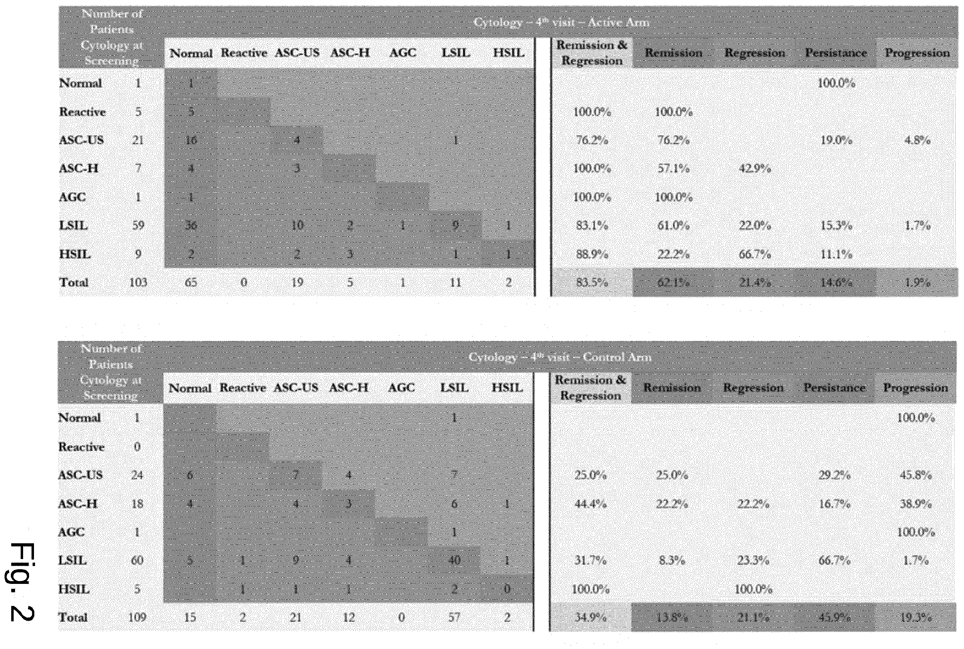

**Fig. 2****: Difference in the cervical smear findings - Screening vs. 4^{th} visit.** Compared to the non-treated control arm the progression rate remained strongly reduced in the active arm treated with the inventive therapy even months after the treatment had ended (1.9% in the active arm vs. 19.3% in the control arm) "at Screening": initial screening of the patient, "at 4^{th} Visit": six months after initial screening.

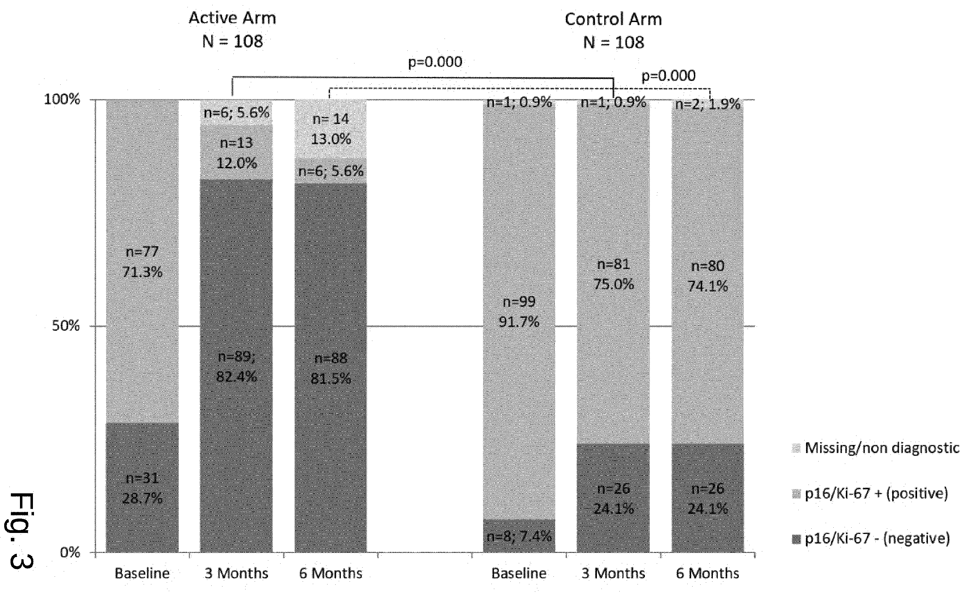

**Fig. 3****: Improvement of p16/Ki-67 status.** A much larger remission rate from p16/Ki-67 double-stain positive was observed in the active arm treated with the inventive therapy, even months after the treatment had ended. "p16/Ki-67 -": negative as determined with the established CINtec^{®} PLUS test (Roche, Switzerland). "p16/Ki-67 +": positive as determined with the established CINtec^{®} PLUS test (Roche, Switzerland).

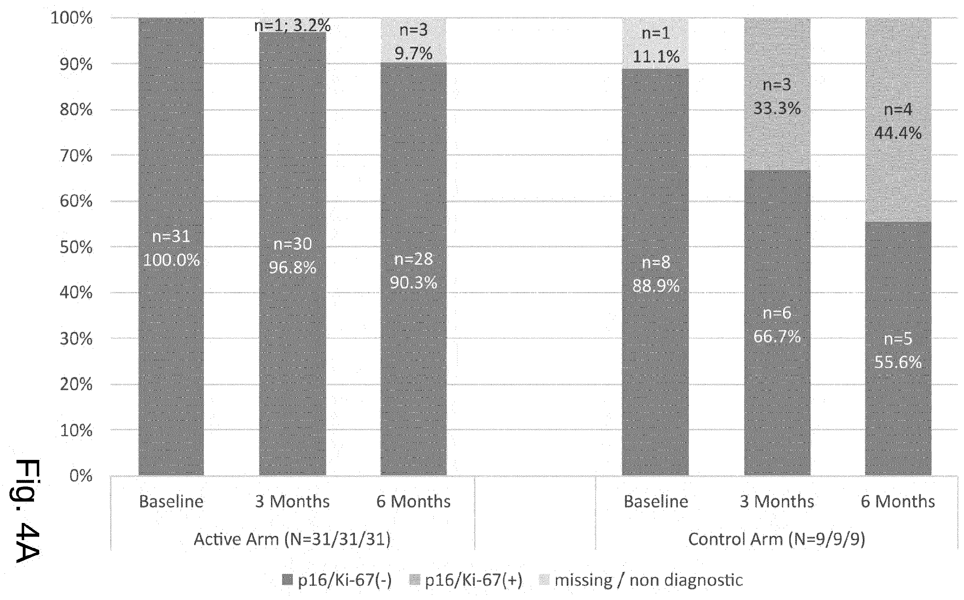

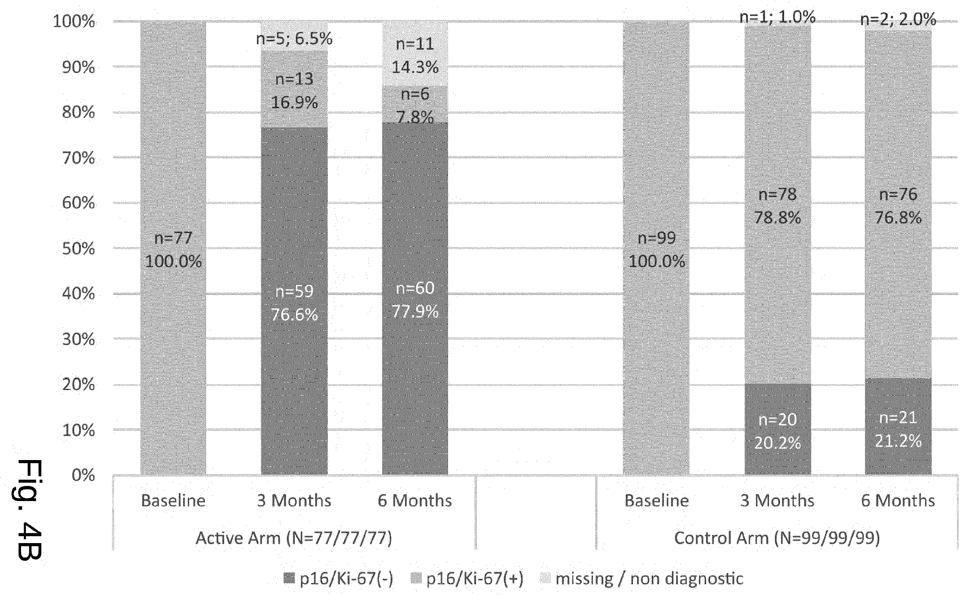

**Figs. 4A** **and** **4B****: Comparison between patients who were p16/Ki-67-negative at baseline (****Fig. 4A****) with patients who were p16/Ki-67-positive at baseline (****Fig. 4B****).** Both groups of patients benefited from the inventive therapy also with respect to p16/Ki-67 status; i.e. by maintaining the status of being p16/Ki-67-negative or by changing the status back to p16/Ki-67-negative. Surprisingly, for the group of patients who were p16/Ki-67-positive at baseline (Fig. 4B), the percentage of patients for whom the inventive therapy achieved a benefit with respect to p16/Ki-67 status during the observation period was much larger, when comparing the respective active arm to the respective control arm. "p16/Ki-67(-)": negative as determined with the established CINtec^{®} PLUS test (Roche, Switzerland). "p16/Ki-67(+)": positive as determined with the established CINtec^{®} PLUS test (Roche, Switzerland).

### Example 1 - Pharmaceutical composition for use in reducing progression of cervical cell abnormalities

The composition is an aqueous vaginal gel having the following ingredients (per 5ml of gel):

| | |
|---|---|
| highly dispersed silicon dioxide | 10.00 mg |
| citric acid | 24.80 mg |
| sodium selenite | 0.83 mg |

(corresponds to 0.25 mg selenium equivalent)

| | |
|---|---|
| sodium benzoate | 2.50 mg |
| potassium sorbate | 5.00 mg |
| hydroxyethyl cellulose | 100.00 mg |
| water | (up to 5 ml) |

### Example 2 - Controlled clinical trial

The present trial was a randomized, prospective, open label with control group multicentre study of 3 x 28 days once a day treatment with the composition of example 1 (female patients, age 25-60 years, no chronic viral disease, cancer and immunosuppressive treatment). Daily dose was 5 ml of the composition, applied intravaginally.

The subjects were recruited in 3 centres. Cervical smears were collected according the standard procedure known in the art, preserved in a specific liquid milieu called SurePath^{®} (Becton & Dickinson) and evaluated in accordance to the Bethesda classification. Samples were further analysed with the CINtec^{®} PLUS cytology kit (Roche, Switzerland).

An interim analysis was conducted after about 50% of patients (N = 111; 50% evaluable per arm) had completed about 3 months in the trial (see Fig. 1). Final evaluations with larger patient sets were conducted at the end of the trial, 3 months after the treatment had ended (see Figs. 2 and 3).

### Results

Evaluated data demonstrate a clearly improved outcome for patients in the active arm for all measured parameters. Only a few slight adverse events were reported.

Compared to the non-treated control arm the progression rate was strongly reduced in the active arm treated with the inventive therapy (see Fig. 1, 0.0% in the active arm vs. 20.7% in the control arm), and remained strongly reduced even months after the treatment had ended (see Fig. 2, 1.9% in the active arm vs. 19.3% in the control arm).

Further, compared to the non-treated control arm, a much larger remission rate from p16/Ki-67 double-stain positive was observed in the active arm treated with the inventive therapy (see Fig. 3).

In addition, for the group of patients who were p16/Ki-67-positive at baseline, the percentage of patients for whom the inventive therapy achieved a benefit with respect to p16/Ki-67 status during the observation period was particularly large (see Figs. 4A and 4B), which came as a surprise.

These results demonstrate the exceptional suitability of the composition for the therapeutic indications mentioned herein.

## Claims

1. A pharmaceutical composition containing a selenite-containing compound and a pharmaceutically acceptable acid, selected from citric acid, acetic acid, malic acid, carbonic acid, sulfuric acid, nitric acid, hydrochloric acid, fruit acids and mixtures thereof, for use in reducing progression of cervical cell abnormalities in a female patient, wherein the patient is p16-positive and Ki-67-positive at least in a region of the cervix uteri, wherein the composition is applied intravaginally.

2. The pharmaceutical composition for use according to claim 1, wherein said cervical cell abnormalities are selected from ASC-US, ASC-H, AGC, LSIL and HSIL, preferably from ASC-US, ASC-H, AGC and LSIL, more preferably from ASC-US, ASC-H and AGC, yet even more preferably from ASC-US and ASC-H, in particular from ASC-US.

3. The pharmaceutical composition for use according to claim 1 or 2, wherein the composition is applied until the patient has become p16-negative, preferably p16-negative and Ki-67-negative, in said region.

4. The pharmaceutical composition for use according to any one of claims 1 to 3, wherein the total selenium content is 0.01 mg - 1.25 mg, preferably 0.025 mg - 1.00 mg, more preferably 0.05 mg - 0.75 mg, even more preferably 0.10 mg - 0.50 mg, yet even more preferably 0.15 mg - 0.40 mg, especially 0.20 mg - 0.30 mg, per 5 ml of the composition; and/or wherein the total selenium dose is 0.01 mg - 1.25 mg, preferably 0.025 mg - 1.00 mg, more preferably 0.05 mg - 0.75 mg, even more preferably 0.10 mg - 0.50 mg, yet even more preferably 0.15 mg - 0.40 mg, especially 0.20 mg - 0.30 mg, per application.

5. The pharmaceutical composition for use according to any one of claims 1 to 4, wherein the composition is applied at least once per day, preferably for at least 30 days, more preferably for at least 60 days, even more preferably for at least 90 days; optionally wherein the application is discontinued during menstruation of the patient.

6. The pharmaceutical composition for use according to any one of claims 1 to 5, wherein the composition is applied once per day, optionally wherein the application is discontinued during menstruation of the patient.

7. The pharmaceutical composition for use according to any one of claims 1 to 6, wherein the application is discontinued during menstruation of the patient; and/or wherein the application is discontinued for at least 30 days, more preferably at least 60 days, even more preferably at least 90 days after treatment for at least 30 days, more preferably for at least 60 days, even more preferably for at least 90 days, optionally wherein the application is discontinued during menstruation of the patient.

8. The pharmaceutical composition for use according to any one of claims 1 to 7, wherein said progression of cervical cell abnormalities comprises at least one of change from ASC-US to ASC-H, change from ASC-US to AGC, change from ASC-US to LSIL, change from ASC-US to HSIL, change from ASC-H to AGC, change from ASC-H to LSIL, change from ASC-H to HSIL, change from AGC to LSIL, change from AGC to HSIL and change from LSIL to HSIL, preferably at least one of change from ASC-US to ASC-H, change from ASC-US to AGC, change from ASC-US to LSIL, change from ASC-US to HSIL, change from ASC-H to AGC, change from ASC-H to LSIL and change from ASC-H to HSIL, more preferably at least one of change from ASC-US to ASC-H, change from ASC-US to AGC, change from ASC-US to LSIL and change from ASC-US to HSIL, in particular at least one of change from ASC-US to ASC-H and change from ASC-US to LSIL.

9. The pharmaceutical composition for use according to any one of claims 1 to 8, wherein the composition is present in the form of a gel, a suspension, an emulsion, a suppository such as gelatine capsules or gelatine-free capsules, a spray or a powder.

10. The pharmaceutical composition for use according to any one of claims 1 to 9, wherein the composition further contains silicon dioxide, preferably highly disperse silicon dioxide.

11. The pharmaceutical composition for use according to any one of claims 1 to 10, wherein the composition has a pH of less than 7.0, preferably less than 5.0, in particular between 4.0 and 2.5.

12. The pharmaceutical composition for use according to any one of claims 1 to 11, wherein the pharmaceutically acceptable acid is citric acid.

13. The pharmaceutical composition for use according to any one of claims 1 to 12, wherein the selenite-containing compound is sodium selenite.

14. The pharmaceutical composition for use according to any one of claims 1 to 13, wherein the patient does not have cancer, and/or chronic viral disease or chronic viral disease other than HPV infection, and/or wherein the patient is not immunosuppressed.

## Patentansprüche

1. Pharmazeutische Zusammensetzung, enthaltend eine Selenit-haltige Verbindung und eine pharmazeutisch verträgliche Säure, ausgewählt aus Citronensäure, Essigsäure, Apfelsäure, Kohlensäure, Schwefelsäure, Salpetersäure, Chlorwasserstoffsäure, Fruchtsäuren und Gemischen davon zur Verwendung bei der Verringerung der Progression von Anomalien der Gebärmutterhalszellen bei einer Patientin, wobei die Patientin mindestens in einem Bereich des Gebärmutterhalses p16-positiv und Ki-67-positiv ist, wobei die Zusammensetzung intravaginal angewendet wird.

2. Pharmazeutische Zusammensetzung zur Anwendung nach Anspruch 1, wobei die Anomalien der Gebärmutterhalszellen aus ASC-US, ASC-H, AGC, LSIL und HSIL, vorzugsweise aus ASC-US, ASC-H, AGC und LSIL, bevorzugter aus ASC-US, ASC-H und AGC, noch bevorzugter aus ASC-US und ASC-H, insbesondere aus ASC-US, ausgewählt sind.

3. Pharmazeutische Zusammensetzung zur Anwendung nach Anspruch 1 oder 2, wobei die Zusammensetzung angewendet wird, bis die Patientin in dem Bereich p16-negativ, vorzugsweise p16-negativ und Ki-67-negativ geworden ist.

4. Pharmazeutische Zusammensetzung zur Anwendung nach einem der Ansprüche 1 bis 3, wobei der Gesamtselengehalt 0,01 mg bis 1,25 mg, vorzugsweise 0,025 mg bis 1,00 mg, bevorzugter 0,05 mg bis 0,75 mg, noch bevorzugter 0,10 mg bis 0,50 mg, sogar noch bevorzugter 0,15 mg bis 0,40 mg, insbesondere 0,20 mg bis 0,30 mg, pro 5 ml der Zusammensetzung beträgt; und/oder wobei die Gesamtselendosis 0,01 mg bis 1,25 mg, vorzugsweise 0,025 mg bis 1,00 mg, bevorzugter 0,05 mg bis 0,75 mg, noch bevorzugter 0,10 mg bis 0,50 mg, sogar noch bevorzugter 0,15 mg bis 0,40 mg, insbesondere 0,20 mg bis 0,30 mg, pro Anwendung beträgt.

5. Pharmazeutische Zusammensetzung zur Anwendung nach einem der Ansprüche 1 bis 4, wobei die Zusammensetzung mindestens einmal pro Tag, vorzugsweise für mindestens 30 Tage, bevorzugter für mindestens 60 Tage, noch bevorzugter für mindestens 90 Tage angewendet wird; wobei die Anwendung gegebenenfalls während der Menstruation der Patientin unterbrochen wird.

6. Pharmazeutische Zusammensetzung zur Anwendung nach einem der Ansprüche 1 bis 5, wobei die Zusammensetzung einmal pro Tag angewendet wird, wobei die Anwendung gegebenenfalls während der Menstruation der Patientin unterbrochen wird.

7. Pharmazeutische Zusammensetzung zur Anwendung nach einem der Ansprüche 1 bis 6, wobei die Anwendung während der Menstruation der Patientin unterbrochen wird; und/oder wobei die Anwendung für mindestens 30 Tage, bevorzugter mindestens 60 Tage, noch bevorzugter mindestens 90 Tage nach der Behandlung für mindestens 30 Tage, bevorzugter für mindestens 60 Tage, noch bevorzugter für mindestens 90 Tage, unterbrochen wird, wobei die Anwendung gegebenenfalls während der Menstruation der Patientin unterbrochen wird.

8. Pharmazeutische Zusammensetzung zur Anwendung nach einem der Ansprüche 1 bis 7, wobei die Progression der Anomalien der Gebärmutterhalszellen mindestens eines von einer Veränderung von ASC-US zu ASC-H, einer Veränderung von ASC-US zu AGC, einer Veränderung von ASC-US zu LSIL, einer Veränderung von ASC-US zu HSIL, einer Veränderung von ASC-H zu LSIL, einer Veränderung von ASC-H zu HSIL, einer Veränderung von AGC zu LSIL, einer Veränderung von AGC zu HSIL und einer Veränderung von LSIL zu HSIL, vorzugsweise mindestens eine von einer Veränderung von ASC-US zu ASC-H, einer Veränderung von ASC-US zu AGC, einer Veränderung von ASC-US zu LSIL und einer Veränderung von ASC-US zu HSIL, insbesondere mindestens eine von einer Veränderung von ASC-US zu ASC-H und einer Veränderung von ASC-US zu LSIL, aufweist.

9. Pharmazeutische Zusammensetzung zur Anwendung nach einem der Ansprüche 1 bis 8, wobei die Zusammensetzung in Form eines Gels, einer Suspension, einer Emulsion, eines Suppositoriums, wie Gelatinekapseln oder gelatinefreien Kapseln, eines Sprays oder eines Pulvers vorliegt.

10. Zusammensetzung zur Anwendung nach einem der Ansprüche von Anspruch 1 bis 9, wobei die Zusammensetzung ferner Siliciumdioxid, vorzugsweise hochdisperses Siliciumdioxid, enthält.

11. Pharmazeutische Zusammensetzung zur Anwendung nach einem der Ansprüche 1 bis 10, wobei die Zusammensetzung einen pH-Wert von weniger als 7,0, vorzugsweise weniger als 5,0, insbesondere zwischen 4,0 und 2,5 hat.

12. Pharmazeutische Zusammensetzung zur Anwendung nach einem der Ansprüche 1 bis 11, wobei die pharmazeutisch verträgliche Säure Citronensäure ist.

13. Pharmazeutische Zusammensetzung zur Anwendung nach einem der Ansprüche 1 bis 12, wobei die Selenit-haltige Verbindung Natriumselenit ist.

14. Pharmazeutische Zusammensetzung zur Anwendung nach einem der Ansprüche 1 bis 13, wobei die Patientin keinen Krebs und/oder keine chronische Viruserkrankung oder keine andere chronische Viruserkrankung als eine HPV-Infektion hat und/oder wobei die Patientin nicht immunsupprimiert ist.

## Revendications

1. Composition pharmaceutique contenant un composé contenant un sélénite et un acide pharmaceutiquement acceptable, choisi parmi l'acide citrique, acide acétique, acide malique, acide carbonique, acide sulfurique, acide nitrique, acide chlorhydrique, acides de fruit et mélanges de ceux-ci, pour une utilisation dans la réduction de la progression d'anomalies de cellules cervicales chez un patient femme, dans laquelle la patiente est p16-positive et Ki-67-positive dans au moins une région du col de l'utérus, dans laquelle la composition est appliquée de manière intravaginale.

2. Composition pharmaceutique pour une utilisation selon la revendication 1, dans laquelle lesdites anomalies de cellules cervicales sont choisies parmi ASC-US, ASC-H, AGC, LSIL et HSIL, de préférence parmi ASC-US, ASC-H, AGC et LSIL, encore mieux parmi ASC-US, ASC-H et AGC, bien mieux encore parmi ASC-US et ASC-H, en particulier ASC-US.

3. Composition pharmaceutique pour une utilisation selon la revendication 1 ou 2, dans laquelle la composition est appliquée jusqu'à ce que la patiente devienne p16-négative, de préférence p16-négative et Ki-67-négative dans ladite région.

4. Composition pharmaceutique pour une utilisation selon l'une quelconque des revendications 1 à 3, dans laquelle la teneur totale en sélénium est de 0,01 mg - 1,25 mg, de préférence 0,025 mg - 1,00 mg, encore mieux 0,05 mg - 0,75 mg, bien mieux encore 0,10 mg - 0,50 mg, particulièrement bien mieux encore 0,15 mg - 0,40 mg, spécialement 0,20 mg - 0,30 mg, pour 5 ml de la composition ; et/ou dans laquelle la dose totale de sélénium est de 0,01 mg - 1,25 mg, de préférence 0,025 mg - 1,00 mg, encore mieux 0,05 mg - 0,75 mg, bien mieux encore 0,10 mg - 0,50 mg, particulièrement bien mieux encore 0,15 mg - 0,40 mg, spécialement 0,20 mg - 0,30 mg, par application.

5. Composition pharmaceutique pour une utilisation selon l'une quelconque des revendications 1 à 4, dans laquelle la composition est appliquée au moins une fois par jour, de préférence pendant au moins 30 jours, encore mieux pendant au moins 60 jours, bien mieux encore pendant au moins 90 jours ; éventuellement dans laquelle l'application est interrompue pendant la menstruation de la patiente.

6. Composition pharmaceutique pour une utilisation selon l'une quelconque des revendications 1 à 5, dans laquelle la composition est appliquée une fois par jour, éventuellement dans laquelle l'application est interrompue pendant la menstruation de la patiente.

7. Composition pharmaceutique pour une utilisation selon l'une quelconque des revendications 1 à 6, dans laquelle l'application est interrompue pendant la menstruation de la patiente ; et/ou dans laquelle l'application est interrompue pendant au moins 30 jours, encore mieux au moins 60 jours, bien mieux encore au moins 90 jours après le traitement pendant au moins 30 jours, encore mieux pendant au moins 60 jours, bien mieux encore pendant au moins 90 jours, éventuellement dans laquelle l'application est interrompue pendant la menstruation de la patiente.

8. Composition pharmaceutique pour une utilisation selon l'une quelconque des revendications 1 à 7, dans laquelle ladite progression d'anomalies de cellules cervicales comprend au moins une de modification de ASC-US en ASC-H, modification de ASC-US en AGC, modification de ASC-US en LSIL, modification de ASC-US en HSIL, modification de ASC-H en AGC, modification de ASC-H en LSIL, modification de ASC-H en HSIL, modification de AGC en LSIL, modification de AGC en HSIL et modification de LSIL en HSIL, de préférence au moins une de modification de ASC-US en ASC-H, modification de ASC-US en AGC, modification de ASC-US en LSIL, modification de ASC-US en HSIL, modification de ASC-H en AGC, modification de ASC-H en LSIL et modification de ASC-H en HSIL, encore mieux au moins une de modification de ASC-US en ASC-H, modification de ASC-US en AGC, modification de ASC-US en LSIL et modification de ASC-US en HSIL, en particulier au moins une de modification de ASC-US en ASC-H et modification de ASC-US en LSIL.

9. Composition pharmaceutique pour une utilisation selon l'une quelconque des revendications 1 à 8, dans laquelle la composition est présente dans la forme d'un gel, une suspension, une émulsion, un suppositoire comme des capsules en gélatine ou des capsules exemptes de gélatine, une pulvérisation ou une poudre.

10. Composition pharmaceutique pour une utilisation selon l'une quelconque des revendications 1 à 9, dans laquelle la composition contient de plus du dioxyde de silicium, de préférence dioxyde de silicium hautement dispersé.

11. Composition pharmaceutique pour une utilisation selon l'une quelconque des revendications 1 à 10, dans laquelle la composition présente un pH inférieur à 7,0, de préférence inférieur à 5,0, en particulier de 4,0 à 2,5.

12. Composition pharmaceutique pour une utilisation selon l'une quelconque des revendications 1 à 11, dans laquelle l'acide pharmaceutiquement acceptable est l'acide citrique.

13. Composition pharmaceutique pour une utilisation selon l'une quelconque des revendications 1 à 12, dans laquelle le composé contenant un sélénite est le sélénite de sodium.

14. Composition pharmaceutique pour une utilisation selon l'une quelconque des revendications 1 à 13, dans laquelle la patiente ne présente pas de cancer et/ou de maladie virale chronique ou maladie virale chronique autre qu'une infection par VPH, et/ou dans laquelle la patiente n'est pas immunodéprimée.
